(19) Europäisches Patentamt

European Patent Office

Office européen des brevets

(11) **EP 0 862 402 B1**

(12) **EUROPEAN PATENT SPECIFICATION**

(45) Date of publication and mention
of the grant of the patent:
**19.12.2001   Bulletin 2001/51**

(21) Application number: **96936171.6**

(22) Date of filing: **04.10.1996**

(51) Int Cl.⁷: $A61F\ 13/15$

(86) International application number:
**PCT/US96/15885**

(87) International publication number:
**WO 97/18784 (29.05.1997 Gazette 1997/23)**

(54) **WATER DISPERSIBLE AND FLUSHABLE ABSORBENT ARTICLE**

IN WASSER DISPERGIERBARER UND WEGSPÜLBARER ABSORBIERENDER ARTIKEL

ARTICLE ABSORBANT JETABLE DANS LES TOILETTES ET SE DISPERSANT DANS L'EAU

(84) Designated Contracting States:
**BE DE ES FR GB GR IT NL SE**

(30) Priority: **22.11.1995  US 561720**
**22.11.1995  US 561989**
**22.11.1995  US 561721**

(43) Date of publication of application:
**09.09.1998   Bulletin 1998/37**

(73) Proprietor: **THE PROCTER & GAMBLE COMPANY**
**Cincinnati, Ohio 45202 (US)**

(72) Inventors:
• **CHRISTON, Patricia, Lee**
**Cincinnati, OH 45237 (US)**

• **AHR, Nicholas, Albert**
**Cincinnati, OH 45247 (US)**
• **DIRK, Raymond, John**
**Cleves, OH 45002 (US)**

(74) Representative: **Hirsch, Uwe Thomas et al**
**Procter & Gamble European Service GmbH,**
**Sulzbacher Strasse 40-50**
**65824 Schwalbach am Taunus (DE)**

(56) References cited:
**WO-A-90/03156          WO-A-92/02199**
**WO-A-93/09740          US-A- 3 913 579**
**US-A- 4 514 345          US-A- 5 300 358**

**Description**

FIELD OF THE INVENTION

The present invention is directed to an absorbent article such as a sanitary napkin, panty liner, incontinence pad, or the like. More particularly, the present invention is directed to absorbent articles that may be disposed of during the flush cycle of a conventional toilet without causing disposal problems thereby.

BACKGROUND OF THE INVENTION

Disposable absorbent articles have been commercially available for many years and have met with great success world wide. A long felt need has existed for more convenient and discrete disposal methods for such absorbent articles, particularly for sanitary napkins and the like. One method that would provide such improved convenience and discretion would be the use of a conventional toilet for such disposal. However, there is a substantial risk of disposal problems, such as clogging, if commercially available sanitary napkins are disposed of by flushing them down a conventional toilet.

In the past a number of attempts have been made to provide flushable absorbent articles. British Patent No. 282,447 attempts a partial solution by providing a core said to be flushable and a repellent treated barrier which is separated from the core and needs to be disposed of by other means. U.S. Patent No. 3,078,849, issued to Morse on February 26, 1962, describes a sanitary napkin incorporating a fluid sensitive, temporary barrier within the absorbent core for spreading bodily fluids but makes no provision for a water sensitive outer covering. U.S. Patent No. 3,561,447, issued to Alexander on March 13, 1969, describes a sanitary napkin having a nonwoven fabric covering wherein the nonwoven fabric comprises textile length fibers and the binder for the nonwoven is a combination of a soft acrylic binder and polyvinyl alcohol. This combination is said to have sufficient strength when damp to serve as an outer covering while still dispersing in water when exposed to mild agitation. While such a structure may have limited wet strength, it is unlikely that it will have sufficient barrier properties to be a satisfactory backsheet for a modern sanitary napkin. U. S. Patent 3,665,923, issued to Champaigne, Jr. on May 30, 1972, describes a sanitary napkin with an wrapper comprising a nonwoven fiber web that is bonded by a water dispersible adhesive such as poly (vinyl alcohol). A preferred embodiment also comprises a baffle member of a thin impervious plastic film interposed between the absorbent pad and the wrapper. This structure solves the problem of providing barrier properties by providing a non dispersible member with the requisite barrier properties. Repeated flushing of such structures poses the risk of clogging sewer pipes because the baffle member will not disperse into small particles in a toilet. U.S. Patent 5,300,358, issued to Evers on April 5, 1994 describes the absorbent structures wherein the backsheet comprises two sheets of poly (vinyl alcohol) film with a highly absorbent paper structure therebetween. All surfaces that may be exposed to aqueous fluids are treated with a water repellent material, such as a fluorocarbon. The absorbent structure is also provided with a tear strip or string which, when pulled at disposal, is said to expose the highly absorbent paper structure to water which then wicks the water to the non repellent treated surfaces so they can dissolve. The requirement of a tear strip is an obvious inconvenience.

Thus, it is an object of the present invention to provide an absorbent article with performance properties (such as comfort to a wearer, leakage resistance, and the like) equaling or exceeding those of contemporary absorbent articles. It a further object of the present invention to provide absorbent articles that provide improved convenience and discretion when the used absorbent article is disposed of. It is still a further object of the present invention to provide an absorbent article that may be disposed of by flushing the article down a conventional toilet and which readily disperses into small portions when the used absorbent article is exposed to water and the mixing action of a conventional toilet.

SUMMARY OF THE INVENTION

The present invention provides an absorbent article, such as a sanitary napkin, that disperses into fragments which are readily flushable in a normal toilet. The sanitary napkin of the present invention comprises a liquid pervious topsheet, a backsheet impervious to bodily fluids, an absorbent core positioned between the topsheet and the backsheet, and further technical features as defined in Claim 1.

The preferred liquid pervious topsheet of the present invention comprises a wet laid apertured tissue having a temporary wet strength resin incorporated therein. Portions of the body surface of the tissue are further provided with a resinous material. Preferably, the resinous material comprises a water resistant resinous material that is provided in the form of fibrils printed on the body surface of the topsheet. Alternatively, the resinous material can provide the topsheet with a surface energy gradient between the body surface thereof and the garment surface thereof. The preferred topsheet of the present invention acquires bodily fluids at an excellent rate and serves to prevent such acquired fluids from rewetting the body surface thereof so the sanitary napkin of the present invention has a comfortable feel when it is worn.

The preferred backsheet of the present invention comprises a wet laid fibrous assembly having a temporary wet strength resin incorporated therein. The backsheet is further coated with a water resistant resinous material that causes the backsheet to become impervious to bodily fluids without impairing the spreading of adhesive materials thereon. Backsheets of the type described herein represent an improvement over those described in the art in that flushable absorbent articles of the prior art typically use materials having a very low critical surface tension to help ensure the backsheet would be impervious with resulting difficulty in adhesively joining such backsheets to the remaining components of a sanitary napkin. The backsheet of the present invention presents no such joinder issues.

The sanitary napkin is assembled by disposing the backsheet such that the surface thereof that is coated with the water resistant resinous material is oriented toward the core. The core and the topsheet are disposed thereon, and the components joined using means known to those skilled in the art. A water soluble adhesive is used to join the components of the preferred sanitary napkin of the present invention in at least an area of peripheral bonding so the components will separate when the sanitary napkin is exposed to water in a toilet.

BRIEF DESCRIPTION OF THE DRAWINGS

While the specification concludes with claims particularly pointing out and distinctly claiming the subject matter which is regarded as forming the present invention, it is believed that the invention will be better understood from the following description taken in conjunction with the accompanying drawings, in which:

Figure 1 is a top plan view of a preferred sanitary napkin embodiment of the present invention shown with a portion of the topsheet removed to show the underlying structure;

Figure 2 is an enlarged cross-sectional view of the preferred sanitary napkin embodiment shown in Figure 1 taken along line 2-2 of Figure 1;

Figure 3 is a schematic side elevational view of an apparatus suitable for coating the backsheet or printing the topsheet of the present invention;

Figure 4 is a schematic side elevational view of an alternative apparatus suitable for coating the backsheet or printing the topsheet of the present invention on both sides of the web;

Figure 5 is a plan view of the topsheet of the present invention;

Figure 6 is an enlarged cross-sectional view taken along line 5-5 of the topsheet of the present invention;

Figure 7 is an enlarged cross-sectional view similar to that of Figure 6 but of an alternative web configuration;

Figure 8 is an enlarged cross-sectional view similar to that of Figure 6 but of a further alternative web configuration;

Figure 9 is a greatly enlarged, perspective illustration of an individual fibril 54 such as depicted in Figures 6-8;

Figure 10 is a plan view of an apparatus suitable for flushability determination according to the method described in the TEST METHODS section below;

Figure 11 is a cross section of the flushability apparatus of Figure 10 taken along line 8-8 thereof; and

Figure 12 is a perspective view showing the assembly of the apparatus used to measure hydrostatic head.

DETAILED DESCRIPTION OF THE INVENTION

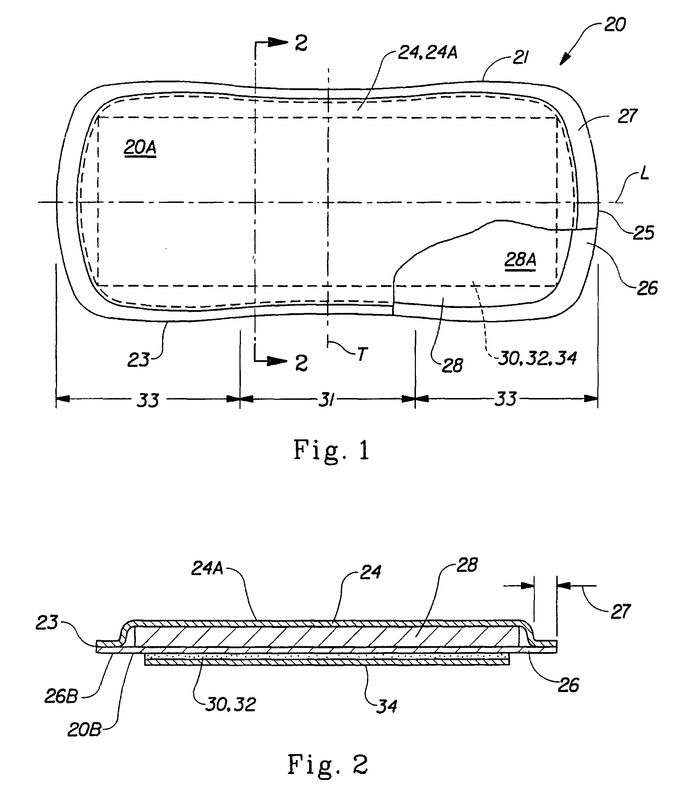

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use, and, preferably, to be recycled, composted or otherwise disposed of in an environmentally compatible manner). A "unitary" absorbent article refers to absorbent articles which are formed of separate parts united together to form a coordinated entity so that they do not require separate manipulative parts like a separate holder and pad. A preferred embodiment of a unitary disposable absorbent article of the present invention is the catamenial pad, sanitary napkin 20, shown in Figure 1. As used herein, the term "sanitary napkin" refers to an absorbent article which is worn by females adjacent to the pudendal region, generally external to the urogenital region, and which is intended to absorb and contain menstrual fluids and other vaginal discharges from the wearer's body (e. g., blood, menses, and urine). Interlabial devices which reside partially within and partially external of the wearer's vestibule are also within the scope of this invention. As used herein, the term "pudendal" refers to the externally visible female genitalia. It should be understood, however, that the present invention is also applicable to other feminine hygiene or catamenial pads such as pantiliners, or other absorbent articles such as diapers, training pants, incontinence pads, and the like.

General Description of the Sanitary Napkin of the Present Invention

Figure 1 is a plan view of the sanitary napkin 20 of the present invention in its flat-out state with portions of the structure being cut-away to more clearly show the construction of the sanitary napkin 20 and with the portion of the sanitary napkin 20 which faces or contacts the wearer, oriented towards the viewer. As shown in Figure 1, the sanitary napkin 20 preferably comprises a liquid pervious topsheet 24, a liquid impervious backsheet 26 joined with the topsheet 24, an absorbent core 28 positioned between the topsheet 24 and the backsheet 26, and attachment means 30 for releasably attaching the sanitary napkin 20 to a wearer's undergarment.

The sanitary napkin 20 has two surfaces, a body-contacting surface or "body surface" 20A and a garment surface 20B. In a similar manner each component comprising the sanitary napkin 20 may have a body surface designated by the reference number for the component with an appended A and a garment surface designated by the reference number for the component and an appended B. The sanitary napkin 20 is shown in Figure 1 as viewed from its body surface. The body surface 20A is intended to be worn adjacent to the body of the wearer while the garment surface 20B is on the opposite side and is intended to be placed adjacent to the wearer's undergarments when the sanitary napkin 20 is worn. The sanitary napkin 20 has two centerlines, a longitudinal centerline L and a transverse centerline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" as used herein, are interchangeable, and refer to a line, axis or direction which lies within the plane of the sanitary napkin 20 that is generally perpendicular to the longitudinal direction.

Figure 1 also shows that the sanitary napkin 20 has a periphery 21 which is defined by the outer edges of the sanitary napkin 20 in which the longitudinal edges are designated 23 and the end edges are designated 25. A central region 31 is disposed between two end regions 33. The end regions 33 preferably extend longitudinally outwardly from the edges of the central region 31 about 12% to about 33% of the length of the sanitary napkin. A detailed description of a sanitary napkin having a central region 31 and the two end regions 33 is contained in U.S. Patent 4,690,680, issued to Higgins on September 1, 1987.

While the topsheet, the backsheet, and the absorbent core may be assembled in a variety of well known configurations (including so called "tube" products or side flap products), preferred sanitary napkin configurations are described generally in U.S. Patent 4,950,264, issued to Osborn on August 21, 1990; U.S. Patent 4,425,130, issued to Desmarais on January 10, 1984; U.S. Patent 4,321,924, issued to Ahr on March 30, 1982; U.S. Patent 4,589,876, issued to Van Tilburg on August 18, 1987. Figure 1 shows a preferred embodiment of the sanitary napkin 20 in which the topsheet 24 and the backsheet 26 have length and width dimensions generally larger than those of the absorbent core 28. The topsheet 24 and the backsheet 26 extend beyond the edges of the absorbent core 28 to thereby form at least a portion of the periphery 21.

The Absorbent Core

The absorbent core 28 may be any absorbent means which is capable of absorbing or retaining liquids (e.g., menses and/or urine). As shown in Figure 1, the absorbent core 28 has a body surface, a garment surface, side edges, and end edges. The absorbent core 28 may be manufactured in a wide variety of sizes and shapes (e.g., rectangular, oval, hourglass, dog bone, asymmetric, etc.) and from a wide variety of liquid-absorbent materials commonly used in sanitary napkins and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these. The configuration and construction of the absorbent core may also be varied (e.g., the absorbent core may have varying caliper zones (e.g., profiled so as to be thicker in the center), hydrophilic gradients, superabsorbent gradients, or lower density and lower average basis weight acquisition zones; or may comprise one or more layers or structures). The total absorbent capacity of the absorbent core should, however, be compatible with the design loading and the intended use of the sanitary napkin. Further, the size and absorbent capacity of the absorbent core may be varied to accommodate different uses such as incontinence pads, pantiliners, regular sanitary napkins, or overnight sanitary napkins.

Exemplary absorbent structures for use as the absorbent core of the present invention are described in U.S. Patent 4,950,264, issued to Osborn on August 21, 1990; U.S. Patent 4,610,678, issued to Weisman et al. on September 9, 1986; U.S. Patent 4,834,735, issued to Alemany et al. on May 30, 1989; and European Patent Application No. 0 198 683, The Procter & Gamble Company, published October 22, 1986 in the name of Duenk, et al. A preferred embodiment of the absorbent core comprises a blend of comminuted wood pulp and a superabsorbent polymer. A wood pulp suitable for comminution into airfelt is provided by the Buckeye Cellulose Corp. of Memphis, TN under the designation Foley Fluff. A suitable superabsorbent polymer is provided by Nalco Chemical Co. of Naperville, IL under the designation Nalco 1180.

The Backsheet

The backsheet 26 is impervious to bodily fluids (e.g., menses and/or urine) yet readily dispersible in cold water under the mild agitation seen when flushing a conventional toilet. As used herein, a material is impervious to bodily fluids (i. e. "water resistant") if the material is capable of capable of maintaining a hydrostatic head that is greater than about 12 cm without substantial leakage when evaluated using the method described in the TEST METHODS section below. The backsheet 26 is preferably manufactured from a wet laid tissue that also comprises a temporary wet strength resin. The tissue has also been coated with a water resistant resinous material. The backsheet preferably has a matte finish to provide a more clothlike appearance. Further, the backsheet 26 may permit vapors to escape from the absorbent core 28 (i.e., breathable) while still preventing exudates from passing through the backsheet 26.

While a coated, wet laid tissue is preferred for the present invention, any fibrous assembly that is impervious to bodily fluids, yet readily dispersible in cold water under mild agitation is suitable. Thus, suitable materials include carded, air laid, or wet laid assemblies of hydrophilic fibers. Suitable fibers include, but are not limited to, natural fibers (e.g., wood or cotton fibers), synthetic fibers (e.g., polymeric fibers such as polyester, nylon, viscose rayon fibers, cellulose acetate, polypropylene, or polyethylene fibers) or a combination of natural and synthetic fibers. The substrate 102 may also at least partially comprise chemically modified natural fibers, such as cross-linked cellulose fibers. Suitable cross-linked cellulose fibers are described in U.S. Patent No. 4,888,093 issued Dec. 19, 1989 to Cook, et al.; U. S. Patent No. 4,822,543 issued Apr. 18, 1989 to Dean, et al.; U.S. Patent No. 4,898,642 issued Feb. 6, 1990 to Moore, et al.; U.S. Patent No. 4,935,022 issued June 6, 1990 to Lash, et al.; U.S. Patent No. 5,137,537 issued Aug. 11, 1992 and U.S. Patent No. 5,183,707 issued February 2, 1993 to Herron et al. In order to ensure easy dispersibility, such fibers should be either intrinsically hydrophilic or treated to be hydrophilic. As used herein, the contact angle between water and the material surface is used to define its relative hydrophilicity. The contact angle is less than 90 degrees for a material to be considered to be a "hydrophilic" material. Methods of treating fibrous assemblies to render them hydrophilic are described in U.S. Patent 4,950,254 issued to Osborn on August 21, 1990.

High internal phase emulsion (HIPE) foams, as described in U.S. Patent 5,147,345, issued to Young, et al. on September 15, 1992, are also suitable for use as the substrate 102. HIPE foams provide the additional advantage that they are also capable of absorbing bodily fluids, and can provide at least a portion of the absorbent capacity of the sanitary napkin.

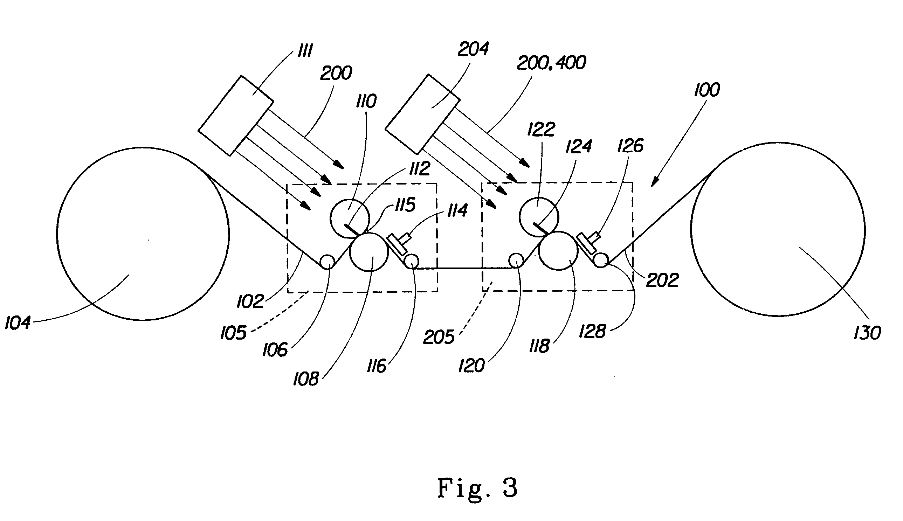

As noted above, the backsheet 26 comprises a tissue coated with a water resistant resinous material. Referring to Figure 3, a process for producing a liquid impermeable and flushable web that is suitable for use as backsheet 26 comprises the following steps. Substrate 102, having a first side and a mutually opposed second side, is unwound from a parent roll 104. The substrate 102 is fed to a first printing station 105 comprising a first backing surface 108, a first printing roll 110, and a first feed roll 106. The first backing surface 108 is positioned opposite the first printing roll 110 to form a first nip therebetween. The first printing roll 110 has a plurality of cells disposed about its periphery. In a preferred embodiment, the first backing surface 108 comprises a first backing roll rotatable about its longitudinal axis. After being heated to the melting point, or otherwise treated, the resinous material 200 is delivered to the first printing roll 110 by a first delivery mechanism 111. The resinous material 200 is then applied to and impregnated with the substrate 102.

Tension control devices and tracking devices (not shown) well known in the art may also be used in this process, if such devices are necessary to insure an even coating of the resinous material 200 across the entire transverse width of the substrate 102.

The water resistant resinous material 200 is provided to first printing roll 110 in a liquid state. If the resinous material 200 is in a solid state at room temperature, it may be heated to a temperature above its melting point. Alternatively, resinous materials in a liquid state at room temperature may also be used. For example, prepolymers comprising chemically reactive end groups may be utilized. In this case, after depositing the resinous material 200 onto and into the substrate 102, a subsequent curing step is necessary to convert a resinous material 200, such as a liquid prepolymer, into the solid state. For example, prepolymers that are curable upon exposure to actinic radiation are suitable.

In the process, first resinous material 200 is heated to at least its melting point, causing the resinous material 200 to be flowable.

The first printing roll 110 rotates about its first longitudinal axis to provide a first peripheral velocity. The substrate 102 is transported, at a transport velocity, relative to the first printing roll 110 and the first backing surface 108, through the first nip defined by the first printing roll 110 and the first backing surface 108. The flowable first resinous material 200 is disposed into the cells of the first printing roll 110. The substrate 102 is transported through the first nip in contacting relation with the cells of the first printing roll 11, thereby applying the flowable first resinous material 200 from the cells of the first printing roll 110 onto the substrate 1020.

While rotary printing processes are well known in the art, the process described and claimed herein differs from such known processes in that during the process of applying the flowable first resinous material 200 onto the substrate 102 according to the present invention, a first velocity differential occurs between the transport velocity of the transported substrate 102 and the peripheral velocity of the first printing roll 110. The first peripheral velocity of the first printing roll 110 is at least about 100% greater than the transport velocity of the substrate 102, so that a first velocity differential occurs. The term "velocity differential" is defined herein as:

$$\text{(peripheral velocity - transport velocity)/transport velocity} \times 100\%.$$

Preferably, the first velocity differential ranges from about 100% to about 500%. More preferably, the first velocity differential ranges from 300% to 350%.

This first velocity differential causes the first printing roll 110 to wipe the first resinous material 200 onto and into the substrate 102 in a shearing action and thereby creates an even and uniform application of the first resinous material 200 therein, such that the resinous material 200 penetrates the substrate 102 and occupies the interstitial spaces and voids of the substrate 102. Such wiping application under shear stress prevents the caliper of the substrate from increasing when the first resinous material 200 is applied thereto. Caliper is measured with a caliper gauge, Model 65.503, supplied by Ono Sokki Co. of Japan, using a 0.95 inch (2.4 cm) diameter presser foot and a confining pressure of 0.1 psi (0.7 kPa).

As noted above, the wiping process created by the first velocity differential causes the first resinous material 200 to penetrate deeper into the substrate 102 than a conventional rotary printing process allows. By way of non-limiting example, a through air dried paper having apertures measuring 3.5 millimeters x 1.5 millimeters and a caliper of 0.5 millimeters was impregnated with resin according to the present invention as described below. After the doctoring process, the resin in the apertures was measured to have a thickness ranging from 0.10 millimeters to 0.34 millimeters. Interestingly, the resinous material 200 occurred on the face of the coated web 202 contacting the backing surface 108. The first side of the coated web 202 oriented towards the printing roll 110 and doctor blade 112 was generally free of the resinous material 200. The depth of the resin was measured, for this example, using a digital Vernier micrometer having pointed opposing tips with a footprint of less than 1 square millimeter. One of the tips rested on a balance so the confining force could be measured. The thickness of the resinous material 200 in the apertures of the coated web 202 was measured at a confining force between 0.01 and 1.00 grams. These readings were confirmed with a non-contacting laser displacement center having a visible beam measuring 1 millimeter x 2 millimeters.

Such penetration of the first resinous material 200 into the substrate 102 causes the resulting coated web 202 to become liquid impermeable without increasing the caliper of the substrate. In addition, this penetration of the resinous material 200 into the substrate 102 enhances the tensile, shear, burst and tear strengths of the resulting coated web 202. The tensile strength of the resulting coated web 202 is at least 2 times as great as the tensile strength of a like substrate having the same caliper as the resulting web.

With continuing reference to Figure 3, the process according to the present invention may utilize a variety of types of rolls for printing roll 110, including, but not limited to screen printing rolls and a gravure cylinders. Screen printing is well known in the art, as illustrated by U.S. Patent No. 4,628,857, issued December 16, 1986 to Coningsby. Gravure printing is also well known in the art as illustrated by U.S. Patent No. 4,634,130, issued Feb. 17, 1988, to Sheath, to illustrate the general state of the art.

In a preferred embodiment of the present invention, a screen printing roll is used for printing roll 110. The thickness of the screen is preferably from 0.002 (0.005 cm) to 0.007 (0.007 cm) inches. Preferably, a 50 mesh nero screen is used having 50 x 50 cells per square inch. The cells may measure 0.0035 (0.0089 cm) inches across the flats and be oriented at a 20 degree angle relative to the circumferential direction of the printing roll 110. If desired, a chrome plated honeycomb screen may be utilized for strength, particularly if a fine mesh size is desired.

The portion of printing roll 110 from which resin is applied to the substrate 102 is referred to as the printing zone. If desired, printing roll 110 may have zones which do not print, hereinafter referred to as non-printing zones. The printing zones deposit resinous material 200 onto the substrate 102 only in those regions corresponding to the registration of the printing zones with the substrate 102. Likewise, the areas of the substrate 102 which were registered with the non-printing zones will be relatively, if not completely, free of the resinous material 200.

If a gravure roll is selected for the first printing roll 110, the non-printing zones may simply be smooth areas of the roll which do not contain gravure cells. If a screen printing roll is selected for the first printing roll 110, the screen printing roll may have impermeable bars which block transmission of the first resinous material 200 through the screen at the positions of the bars.

The non-printing zones may be generally longitudinally oriented and parallel to the axis of the printing roll. This arrangement produces generally cross machine direction oriented zones in the substrate 102 which do not contain the first resinous material 200. This arrangement may be utilized, if desired, to provide a web that is suitable for use as a diaper backsheet wherein the non-coated areas would define the waist regions thereof.

Alternatively, the non-printing zones may be generally circumferentially oriented, resulting in machine direction oriented zones of the substrate 102 not having the resinous material 200 printed thereon. If desired, several repeating units of the coated web 202 having zones with and without the resinous material 200 may be juxtaposed together to form a resulting web which is relatively wide in the cross machine direction. The resulting coated web 202 is then slit in the machine direction at positions corresponding to the non-printed zones to produce roll stock for use in later production. This arrangement provides the benefits of economy of scale in making disposable absorbent articles according to the present invention.

A first doctor blade 112 is used to insure that the first resinous material 200 is evenly metered across the entire application face of the printing roll 110. The first doctor blade 112 is positioned in non-contacting relationship with a surface of the first printing roll 110 and held stationary as the printing roll 110 rotates, allowing the first doctor blade 112 to wipe the circumference of the first printing roll 110. The first doctor blade 112 scrapes any of the first resinous material 200 not disposed within the individual cells from the first printing roll 110 and, in so doing helps to insure that all the cells are completely filled.

The first backing surface 108 is smooth and can either comprise a rotatable backing roll or a stationary surface. For the preferred embodiment described herein, the first backing surface 108 comprises a rotatable backing roll. The first printing roll 110 and the first backing surface 108 are compressed against each other to provide sufficient engagement between the substrate 102 and the first resinous material 200 that is in the cells of the first printing roll 110 in order to promote wiping of the first resinous material 200 onto and into the substrate 102.

If a thermoplastic resin is selected as the first resinous material 200, the first printing roll 110 is preferably heated to prevent premature solidification of the flowable first resinous material 200. A printing roll temperature of about 270°F (132°C) has been found to work well with the first resinous material 200 and process conditions described below.

First resinous material 200 may be externally heated by known means (not shown) to maintain the material 200 in a liquid state and at the proper temperature and viscosity. Typically, the first resinous material 200 is maintained at a temperature slightly above the melting point. The temperature is considered to be at or above the melting point if the first resinous material 200 is partially or wholly in the liquid state. If the temperature is too low, first resinous material 200 may not transfer from the printing roll 110 to the substrate 102, or, subsequently, may not be suitable for the wiping process described above. Conversely, if the temperature is too high, first resinous material 200 may not be viscous enough to be suitable for the wiping process or the material may thermally decompose. Further, the resin temperature should not be so high as to damage the substrate 102. A preferred temperature range of the first resinous material 200 is between about 200°F (93°C) and about 250°F (121°C) at the point of application to the substrate 102. This temperature is above the melting point of the preferred CA-105 resin (discussed below) but below the temperature where melt viscosity is unsatisfactorily low.

Cooling air may optionally be provided from a first air cooling system 114. The cooling air may be necessary to insure that the first resinous material 200, already applied to the substrate 102, has solidified before the coated web 202 is further treated as is described below.

In a preferred embodiment of the invention, a two step coating process is utilized. A two step coating process is preferred because it provides greater impermeability of the resulting coated web 202 at a lower coating weight than an equivalent single step coating process. In a two step coating process, the coated web 202 may be fed to a second printing station 205 comprising a second backing surface 118, a second printing roll 122, and a second feed roll 120. A second application of the first resinous material 200 by a second delivery mechanism 204 may then be made using the second printing roll 122 and the second backing surface 118 in substantially the same manner as described above. Alternatively, a second resinous material 400, different from the first resinous material 200, may be used in the second coating process.

The second velocity differential of the second coating step may be equal to, less than or greater than the velocity differential at the first coating step. Preferably, the second velocity differential is equal to the first velocity differential, particularly if the first resinous material 200 is used at both the first and second printing stations 105, 205.

A second cooling air system 126 may also be utilized to insure that the first resinous material 200 or the alternative second resinous material 400 applied to the partially coated substrate 102 at the second coating step has solidified before the coated web 202 is wound into finished roll 130.

The coated web 202 is then removed from the second printing roll 122 by the second stripping roll 128 and wound into a finished roll 130.

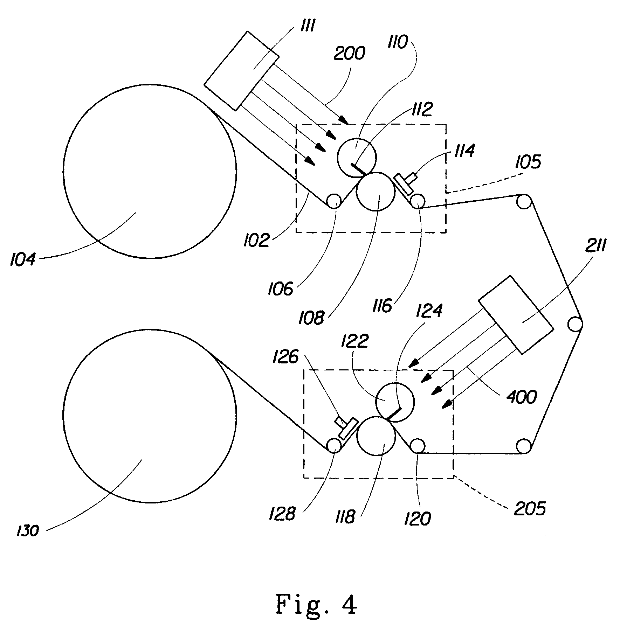

In an alternative embodiment of the invention, shown in Figure 4, the coating applied by the first printing station 105 and the coating applied by the second printing station 205 are applied to the first and second mutually opposed sides of the substrate 102. As can be seen therein, the web path is such that first resinous material 200 is applied by first printing roll 110 to a first side of substrate 102 and second resinous material 400 is applied to the opposed second side of substrate 102 by second printing roll 122. Otherwise, the steps at each print station 105, 205 are substantially the same as those described above.

As noted above, a preferred fibrous assembly suitable for use as substrate 102 is a wet laid tissue having a wet strength resin incorporated therein. A suitable tissue has a basis weight of about 12 pounds per 3000 square feet and is available from Georgia-Pacific Corp. of Bellingham, WA under the designation DST-1. Also as noted above, the wet laid tissue is preferably coated with a water resistant resinous material to render it impermeable to bodily fluids. A water resistant resinous material suitable for use as resinous material 200,400 is a hot melt resin blend which is available from Century International of Columbus, OH under the designation CA-105. Preferably, the coating weight is between about 0.005 grams per square inch (8 grams per square meter) and about 0.075 grams per square inch (116 grams per square meter). More preferably, the coating weight is between about 0.015 grams per square inch (23 grams per square meter) and about 0.035 grams per square inch (54 grams per square meter).

When such a wet laid tissue is coated using the resin application process described above with such a water resistant resinous material, the resulting coated web is impervious to bodily fluids. Specifically, the coated web is capable of maintaining a hydrostatic head of at least about 15 centimeters when tested as described in the TEST METHODS section. Preferably, the coated web is capable of maintaining a hydrostatic head of at least about 18 centimeters.

Not only are the coated webs of the present invention impervious to bodily fluids, they also rapidly lose mechanical integrity and dissociate into fragments on immersion in water. For example, when samples of such coated webs are evaluated for flushability using the method described in the TEST METHODS section, the coated web behaves substantially the same as a sample of a commercially available toilet tissue (CHARMIN® ) used as a control. That is, the sample of the coated web of the present invention breaks up into smaller pieces that readily pass through the test apparatus with no clogging. Reduction in burst strength on exposure to water is one measure of the loss of mechanical integrity discussed above. Table 1 below shows burst strength data for a sample of the preferred backsheet 26 of the present invention.

TABLE 1

| Coated Fibrous Assembly No. | 1 |
|---|---|
| Fibrous Assembly Type | DST-1 |
| Resinous Coating Material | CA-105 |
| Coating Weight | 0.022 g/in$^2$ (g/6.45 cm$^2$) |
| Burst Strength (Grams) | |
| Dry | 1101 |
| Wet (20 Second Soak) | 477 |
| Wet Burst (20 Second)/Dry Burst | 0.43 |

As can be seen, the reduction in burst on exposure to water (forty-three percent of the dry value after 20 seconds) means that the backsheet 26 is sufficiently weakened that it will disperse into fragments under the mild agitation conditions encountered when a conventional toilet is flushed.

The backsheet 26 loses strength primarily because the water resistant resinous material no longer protects the fibrous web from water. As is noted in the Assembly of the Sanitary Napkin section below, the coated fibrous assembly that comprises the preferred backsheet 26 is disposed such that the coated surface comprises the body surface of the backsheet 26. When the sanitary napkin 20 is assembled in this manner, the water resistant resinous material is disposed between the fibrous assembly of the backsheet and those components of the sanitary napkin 20 which are intended to be wet with bodily fluids such as the topsheet 24 and the absorbent core 28. Thus the coating protects the fibrous assembly from absorbed bodily fluids and the fibrous assembly provides the requisite mechanical integrity to the backsheet 26.

Using the water resistant resinous material of the present invention represents an improvement over the hydrophobic materials typically used by the prior art to protect the water sensitive material comprising the backsheet (a typical prior art water sensitive material is poly (vinyl alcohol) and a typical prior art hydrophobic material is a fluorocarbon). Specifically, the hydrophobic materials used by prior art have very low critical surface tensions. For example, the critical surface tension of Teflon® is less than 20 dynes per centimeter (Adamson, A. W., Physical Chemistry of Surfaces, 1976, John Wiley & Sons, New York, page 354). The critical surface tension of other fluorocarbon treated surfaces is similar. This low critical surface tension means that assembly of an absorbent article will be made more difficult because a low critical surface tension interferes with adhesive bonding because adhesives will not spread on and adhere to such surfaces (Low critical surface tension is also the basis of commercially available anti-stain treatments because stains will not adhere to surfaces having a low critical surface tension). This means there is a need to either ensure that there is no fluorocarbon in areas of adhesive bonding (with the resulting manufacturing complexity of insuring adequate registration of those areas with the remaining components of a sanitary napkin) or to treat any fluorocarbon surface in an area of adhesive bonding to increase the critical surface tension thereof. Conversely, a surface coated with the preferred water resistant resinous material of the present invention has a critical surface tension of greater than about 34 dynes per centimeter when measured using the modified TAPPI test method (T 698 pm-83) described in the TEST METHODS section below. Thus, ordinary manufacturing processes can be used to assemble a sanitary napkin using the preferred backsheet of the present invention without the necessity of additional processing steps.

Thus, as used herein, a water resistant resinous material not only provides a fibrous assembly with a surface that is impervious to bodily fluids (i. e., capable of supporting a hydrostatic head of greater than about 12 cm) but also provides the coated web with a surface suitable for joining to other components using adhesive means (i. e., critical surface tension greater than about 34 dynes per centimeter).

The Topsheet

The topsheet 24 is compliant, soft feeling, and non-irritating to the wearer's skin. Further, the topsheet 24 is liquid pervious permitting liquids (e.g., menses and/or urine) to readily penetrate through its thickness. The topsheet should also be readily dispersible under the mild agitation conditions encountered when a conventional toilet is flushed. A suitable topsheet 24 may be manufactured from a wide range of materials such as air laid, wet laid, or carded nonwoven materials. Suitable materials can be comprised of natural fibers (e.g., wood or cotton fibers), synthetic fibers (e.g., polymeric fibers such as polyester, polypropylene, or polyethylene fibers) or from a combination of natural and synthetic fibers.

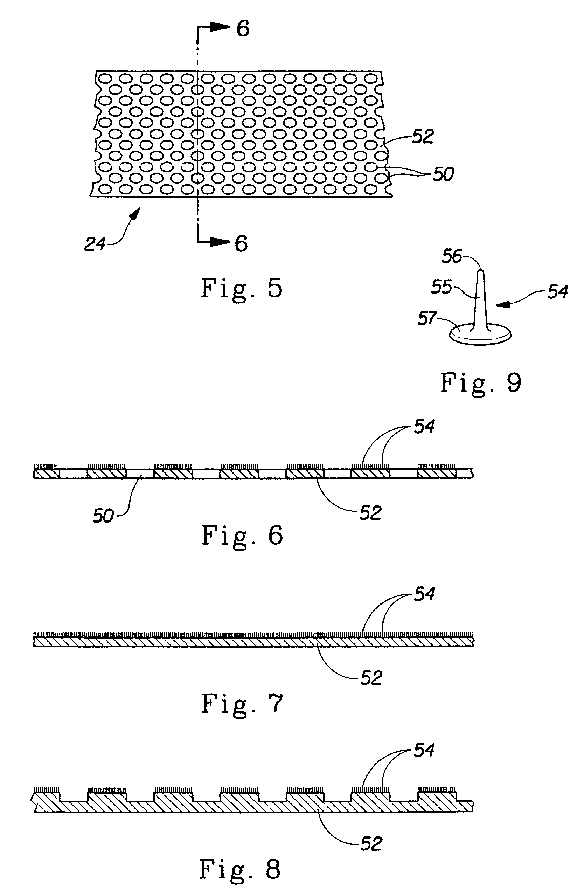

A preferred topsheet 24 comprises a wet laid apertured tissue having a temporary wet strength resin incorporated therein. A portion of such topsheet 24 is shown in Figures 5 and 6. As is shown in Figures 5 and 6, the wet laid tissue that comprises the preferred topsheet 24 comprises a wet laid fibrous assembly 52 having a multiplicity of apertures 50 therethrough. While a preferred fiber furnish for this tissue comprises wood fibers, preferably about 90 percent Eucalyptus fibers and about 10% Northern Sulfite Kraft fibers, other fibrous materials, including but not limited to natural fibers (e.g., other types of wood fibers or cotton fibers), synthetic fibers (e.g., polymeric fibers such as polyester, polypropylene, or polyethylene fibers) or a combination of natural and synthetic fibers are also suitable as long as the fibers are, or can be treated to become, hydrophilic. Such a wet laid apertured tissue can be produced on a paper machine according to the method described in U.S. Patent 3,881,987, issued to Benz on May 6, 1975. Preferably, the drainage member described in the aforementioned Benz patent should comprise the foraminous members described in U.S. Patent 4,514,345, issued to Johnson, et al. on April 30, 1985. The embryonic fibrous webs as would be produced as described above can be further dried using any convenient drying means as would be known to those skilled in the paper making art. For example, press felts, thermal hoods, infrared radiation, blow-through dryers and Yankee drying drums, either used alone or in combination. A particularly preferred drying method uses a press felt and a Yankee drying drum in sequence.

The use of such a method can provide wet laid fibrous assemblies having a range of aperture densities and percent open area. As used herein, the term "aperture density" is intended to mean the number of apertures per square inch of fibrous assembly surface and the term "percent open area" is defined as that portion of the fibrous assembly surface that is not occupied by fibers expressed as a percentage. Preferably the aperture density is between about 9 apertures per square inch (1 aperture per square centimeter) and about 400 apertures per square inch (62 apertures per square centimeter). More preferably the aperture density is between about 20 apertures per square inch (3 apertures per square centimeter) and about 111 apertures per square inch (17 apertures per square centimeter). The preferred apertured wet laid fibrous assemblies of the present invention preferably have a percent open area between about 20 percent and about 50 percent. More preferably the percent open area is between about 30 percent and about 40 percent. A particularly preferred wet laid fibrous assembly has an aperture density of about 81 apertures per square inch (6 apertures per square centimeter) with about 36 percent open area.

The tissue furnish further comprises a temporary wet strength resin. Such a temporary wet strength resin helps the topsheet 24 maintain its mechanical integrity during use of the sanitary napkin 20 yet does not interfere with the dispersibility of the topsheet when the used sanitary napkin 20 is flushed. Suitable temporary wet strength resins are the glyoxalated polyacrylamide resins available from Cytec Industries Inc. of Stanford, CT under the designation Parez™. Particularly preferred is Parez™ 631 NC. When Parez™ 631 NC is used at a level between about 0.5% and about 1.0% in the wet laid apertured tissue, the topsheet 24 has a satisfactory balance of mechanical integrity during use and dispersibility during disposal.

This preferred tissue is further provided with a multiplicity fibrils 54 or "hairs" on the nonapertured portion of its body facing surface. These fibrils 54 reduce the surface wetness characteristics of the topsheet 24 by separating the wearer's body from any bodily fluids that may remain on the cellulosic body side surface of the topsheet 24A. Table 2 compares the surface wetness characteristics of the topsheet 24 to the nonwoven topsheet used on a commercially available sanitary napkin (KOTEX® OVERNITES from Kimberly Clark Corp. Neenah, WI).

TABLE 2

| Topsheet No. | 1 | 2 |
|---|---|---|
| Topsheet Type | Present Invention | Nonwoven |
| Resinous Coating Material | CA-105 | None |
| Fibril Density | 4500 fibrils/in$^2$ [fibrils/6.45 cm$^2$] | N/A |
| Surface Wetness | 0.39g | 0.49g |

As can be seen in Table 2, the preferred topsheet of the present invention has somewhat improved surface wetness when compared to a typical nonwoven topsheet. The fibrils 54 also provide the body surface 24A with a pleasant, velour-like tactile feel.

The fibrils 54 preferably comprise the same water resistant resinous material used to coat the backsheet 26 to render it impermeable to bodily fluids (CA-105). However, the application method described below only applies the water resistant resinous material to portions of the body surface 24A of the topsheet 24. The fibril density can vary between about 500 fibrils per square inch (77 fibrils per square centimeter) to about 11,000 fibrils per square inch (1700 fibrils per square centimeter). Preferably, the fibril density is between about 3000 fibrils per square inch (450 fibrils per square centimeter) and about 5000 fibrils per square inch (775 fibrils per square centimeter). Fibril length can vary between about 0.003 inches (0.07 mm) to about 0.04 inches (1.0 mm). Preferably, the fibril length is between about 0.004 inches (0.1 mm) and about 0.01 inch (0.3 mm). The Applicants have found that choice of fibril length and fibril density allows surface wetness and other topsheet characteristics, including the tactile feel, to be varied to achieve a desired balance of these characteristics.

The preferred method for applying the resinous material is by a rotary screen printing method similar to that described above with respect to the backsheet 26. It is to be understood, however, that other methods of printing or spraying such resinous projections are contemplated by the present invention. Such methods include spiral spraying, mist spraying, or line spraying, gravure printing, and flexographic printing. Rotary screen printing is the most preferred because the method is capable of producing the sizes and densities of fibrils desired and can also be performed at high web line speeds. It should also be noted that a single print station, such as print station 105 as shown in Figure 3, is all that is necessary to provide a substrate 102 with fibrils 54.

The key difference between a method suitable for coating a substrate 102 (see Figure 3) to be suitable for use as a backsheet 26 and the method for coating a substrate 102 to be suitable for use as a topsheet 24 lies in the velocity differential between the transport velocity of the substrate 102 and the tangential velocity of the printing roll 110. Referring again to Figure 3, for a process suitable for coating a substrate 102 for use as a topsheet 24, the transport velocity and the tangential velocity of the printing roll 110 are substantially the same (i. e. the velocity differential is approximately zero). This means that the CA-105 resin is printed on the substrate 102 rather than being wiped across the web surface. By control of the screen pattern on the printing roll 110 and of rheology of the CA-105 when it is in its melted state, the CA-105 is printed as the fibrils 54 described above. Preferably, the printing roll 110 comprises a screen with a hexagonal interlocking mesh pattern having an average aperture diameter of 0.035 (0.0035 cm) inches and the resinous material is maintained at a temperature between about 200°F (93°C) and about 250°F (121°C).

Using the preferred screen printing process described above, the formation of the fibrils can be explained as follows. The apertures in the screen are small enough and the resinous material 200 is of sufficient viscosity that the resinous material will not flow through the apertures in the screen on its own. The doctor blade 112 forces the resinous material 200 to fill up the screen apertures such that a meniscus of the resinous material hangs down from the screen toward the backing surface 108. As the printing roll 110 and backing surface 108 (a roll in the preferred embodiment shown in Figure 3) rotate, the screen contacts the substrate 102 in the nip between the printing roll 110 and the backing surface 108. The meniscus of resinous material 200 transfers from the screen apertures onto the substrate 102. As the screen and printed web separate, cohesive forces within the resinous material and adhesive forces between the resinous material and the screen surface cause a portion of the resinous material 200 to be pulled upward away from the substrate 102 until the separation distance exceeds the elastic limit of the resinous material, causing the material to rupture and form the upper end of the fibril. The point of rupture may be controlled by using a hot wire/hot ribbon 115 placed at a predetermined distance from the printing roll 110 and the backing surface 108, as is known in the art. In this fashion, control of the point of rupture will generate fibrils of the desired caliper and shape.

Figures 7 and 8 depict alternative web structures according to the present invention. As depicted in Figure 7, the fibrous assembly 52 does not include discrete apertures such as apertures 50 depicted in Figures 5 and 6. However, in order to maintain fluid transmission capabilities sufficient to enable their use in absorbent articles, such webs include sufficient inherent porosity. As with the web of Figures 5 and 6, the fibrils 54 are topographically printed upon the uppermost surface of the web rather than only upon the surface areas between apertures. Alternatively, if desired, the area provided with fibrils 54 may be of any desired shape, pattern, or coverage area and of either uniform or non-uniform density.

Figure 8 depicts another embodiment of a fibrous structure 52 according to the present invention. As shown in Figure 8, the web includes regions of various caliper or topography. As with the web of Figure 7, the web lacks discrete apertures but is provided with sufficient inherent porosity to maintain its functionality when included in a disposable absorbent article. As with the webs of Figures 5 and 6, the fibrils 54 are topographically printed upon the uppermost surface of the web rather than the entire upwardly-facing web surface.

With any webs of varying topography, it is to be understood that upon the application of sufficient compressive forces during the application of the fibrils 54, it may indeed be possible to apply fibrils to the entire upwardly-facing web surface due to the web being compressed to a uniform caliper and topography during the printing process.

A wide variety of fibrous webs may be utilized as substrate materials 102 in accordance with the present invention. Suitable materials are discussed above. As noted in that discussion, a preferred substrate material comprises a wet laid, apertured tissue having a temporary wet strength resin incorporated therein.

Figure 9 depicts in much greater detail the physical structure of a typical fibril 54 such as depicted in Figures 6-8. Fibril 54 preferably comprises a shaft portion 55 which extends from a base 57 to a tip 56. In formation, the resin material is deposited as base 57 and drawn upward to form shaft 55. The resin material abruptly separates from the resin remaining on the printing roll 110, forming tip 56, which is preferably slightly rounded. Fibrils are preferably constructed and arranged such that their respective bases 57 are spaced at least slightly apart, such that the underlying substrate therebetween is at least slightly exposed both for acquisition and flushability purposes. In addition, the number and size of the fibrils must not be so great as to likewise impair the flushability and dispersibility of the topsheet.

The resin material utilized to form the fibrils preferably cures or hardens quickly after the fibril has been formed to the desired height and/or configuration. In this fashion, the fibril is "locked in" before the resin can begin to collapse back downward toward the base 57 and the fibril loses its shape. This relatively short cure time, or "open time", is also conducive to comparatively higher web speeds as it ensures the fibers are cured before subsequent web handling operations are undertaken.

If desired, the masking capabilities of the topsheet 24 can be enhanced by the addition of a dye or filler to the resinous material from which the fibrils 54 are made. Suitable masking agents include titanium dioxide and calcium carbonate. The fibrils 54 themselves, as well as the fillers aid in the masking of bodily fluids absorbed by the topsheet and the underlying absorbent core. The masking agent provides a clean and dry appearance by providing additional opacity to the topsheet as a whole, and particularly the upper surface thereof.

Fibrils 54 preferably have sufficient resilience by virtue of material properties, length, and thickness so as to be at least somewhat resistant to deflection toward the web surface when contacted by the wearer in use. If the fibrils collapse too readily, the effect of separation between the wearer and the fibrous hydrophilic substrate is diminished and increasingly "wet" tactile impression may be encountered. Collapsed fibrils if too great in number may also prove to impair fluid acquisition by blocking apertures or pores in the substrate.

Fibrils 54 extend generally outwardly from the body surface of the topsheet 24A, as depicted generally in Figures 6-8. In a preferred configuration, a majority of the fibrils extend generally perpendicularly outwardly from the surface of the web, although at least some of the fibrils may extend outwardly at various angles.

Alternatively, a wet laid apertured tissue produced according to the aforementioned U.S. Patent 3,881,987 on a drainage member as described in the aforementioned U.S. Patent 4,514,345 and having a wet strength resin incorporated therein may further comprise a garment surface 24B. The body surface 24A and the garment surface 24B are separated from one another by an intermediate portion 24C. The wet laid apertured tissue is treated to form a web such that the body surface of the web provides a structure which exhibits a surface energy less than the surface energy of the intermediate portion. In a preferred embodiment, the treated web exhibits a plurality of. regions of comparatively low surface energy which define surface energy gradients where they interface with higher surface energy web surfaces. For example, a silicone resin having a low surface energy can be applied to portions of the body surface 24A providing such regions of comparatively low surface energy. Webs having such surface energy gradients are fully described in PCT Patent application Serial WO 96/00549 in the name of Ouellette.

In a preferred embodiment of the present invention, at least portions of the body surface 24A of the topsheet 24 are hydrophilic so as to help liquid to transfer through the topsheet faster than if the body surface was not hydrophilic. Such a hydrophilic surface helps to diminish the likelihood that bodily fluids will flow off the topsheet rather than flowing into and being absorbed by-the absorbent core. In a preferred embodiment, surfactant is applied to the body surface 24A of the topsheet 24 (e. g. by extrusion coating or spraying) before the fibrils are printed thereon. Alternatively, the body surface of the topsheet can be made hydrophilic by treating it with a surfactant such as is described in the above referenced U.S. Patent 4,950,254.

The Adhesive Member

In use, the sanitary napkin 20 can be held in place by any support means or attachment means 30 well-known for such purposes. Preferably, the sanitary napkin is placed in a wearer's undergarment or panty and secured thereto by a fastener such as an adhesive.

As is shown most clearly in Figure 2, the preferred sanitary napkin 20 of the present invention further comprises an adhesive 32 for attaching the sanitary napkin 20 to a wearer's undergarment. The adhesive 20 provides a means for securing the sanitary napkin in the crotch portion of the panty by releasably adhering the sanitary napkin thereto. Thus, a portion or all of the garment surface 26B of the backsheet 26 is coated with adhesive. As can be seen most clearly in, the adhesive 32 is preferably provided in a rectangular pattern that coats substantially the entire garment surface 26B. It should be noted, however, that other coating patterns are also suitable. Exemplary patterns are described in copending PCT Patent Application WO 92/04000 in the name of Papa, et al..

In keeping with the objects of the present invention, any water sensitive adhesive or glue used in the art for such purposes can be used for the adhesive herein, with pressure-sensitive adhesives being preferred. Suitable adhesives are manufactured by Findley Adhesives Inc. of Wauwatosa, WI. Particularly preferred is the hot melt adhesive available under the designation 9216-02.

In an alternative embodiment of the present invention, the attachment means can comprise the hook member of a hook and loop mechanical fastening system. Again, to be in keeping with the intent of the present invention, it is preferred that such hook attachment means comprise a water sensitive material. It has been found that, the resin (CA-105 from Century Adhesives) that comprises the tibrils 54 of the preferred embodiment of the topsheet 24 is also suitable for use in forming such hook members. Other water sensitive, thermoplastic resinous materials would also be suitable. Methods of producing such hook members and exemplary designs of such members are described in one or more of the following U.S. Patents: 5,058,247, issued to Thomas, et al. on October 22, 1991; 5,116,563, issued to Thomas, et al. on May 26, 1992, and 5,230,851, issued to Thomas on July 27, 1993.

Before the sanitary napkin is placed in use, the pressure-sensitive adhesive is typically covered with a removable release liner 34 in order to keep the adhesive from drying out or adhering to a surface other than the crotch portion of the panty prior to use. Suitable release liners 34 are also described in the above-referenced U.S. Patent 4,917,697. Any commercially available release liners commonly used for such purposes can be utilized herein. Non-limiting examples of suitable release liners are BL30MG-A Silox E1/0 and BL30MG-A Silox 4P/O both of which are manufactured by the Akrosil Corporation of Menasha, WI.

The sanitary napkin 20 of the present invention is used by removing the release liner 34 and disposing thereof in a conventional manner. Thereafter the sanitary napkin is placed in a wearer's panty so that the adhesive 32 contacts the panty. The adhesive 32 maintains the sanitary napkin in its position within the panty during use.

Assembly of the Sanitary Napkin

The topsheet 24 and the backsheet 26 are positioned adjacent the garment surface 28A and the body surface 28B, respectively, of the absorbent core 28 and are preferably joined thereto and to each other by attachment means (not shown) such as those well known in the art. For example, the backsheet 26 and/or the topsheet 24 may be secured to the absorbent core 28 or to each other by a uniform continuous layer of adhesive, a patterned layer of adhesive, or an array of separate lines, spirals, or spots of adhesive. In keeping with the objectives of the present invention, adhesives used in assembling the preferred sanitary napkin 20 should be easily disrupted by the mild agitation conditions encountered when a conventional toilet is flushed. Adhesives which have been found to be satisfactory are the hot melt adhesive available from Findley Adhesives Inc. of Wauwatosa, WI under the designation H-9216-02 and the adhesive emulsion available from Air Products & Chemicals Corp. of Allentown, PA under the designation Airflex 401. Such adhesives may be applied by gravure printing or adhesive sprays. Also suitable is adhesive application by means of an open pattern network of filaments comprising several lines of adhesive filaments swirled into a spiral pattern such as illustrated by the apparatus and method shown in U.S. Patent 3,911,173 issued to Sprague, Jr. on October 7, 1975;

U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, or any other suitable attachment means or combinations of these attachment means as are known in the art.

As noted above and shown in Figures 1 and 2, in the preferred embodiment of the sanitary napkin 20 the topsheet 24 and the backsheet 26 each have length and width dimensions generally larger than those of the absorbent core 28. The topsheet 24 and the backsheet 26 extend beyond the edges of the absorbent core 28 and are joined to each other at least around the periphery 21 in an area of peripheral bonding 27 which is defined by those portions of the topsheet 24 and the backsheet 26 that extend beyond the absorbent core 28. As further noted above, the topsheet 24 is disposed such that the body surface thereof 24A is that surface provided with a multiplicity of fibrils (that is, the fibrils comprise a portion of the outer surface of the sanitary napkin 20), and the backsheet 26 is disposed such that body surface 26A is provided with the moisture resistant resinous coating (that is, the moisture resistant resinous coating is disposed on the interior of the sanitary napkin 20).

In keeping with the objects of the present invention, the topsheet 24 and the backsheet 26 are joined in the area of peripheral bonding 27 by means that are easily disrupted by the mild agitation conditions encountered when a conventional toilet is flushed. Means known to those skilled in the absorbent products art can be used to join the topsheet 24 and the backsheet 26 in the area of peripheral bonding 27 as long as such means do not interfere with the dispersibility of the sanitary napkin 20. Suitable means for joining the topsheet 24 and the backsheet 26 in the area of peripheral bonding 27 are substantially the same as those suitable for joining the topsheet 24 and/or the backsheet 26 to the absorbent core 28. The topsheet 24 and the backsheet 26 are joined using a water soluble adhesive. Suitable adhesives include the hot melt adhesive available from Findley Adhesives Inc. of Wauwatosa, WI under the designation H-9216-02. A preferred water soluble adhesive is the adhesive emulsion which is available from Air Products & Chemicals Corp. of Allentown, PA as Airflex 401.

Depending on the form of the adhesive, the water soluble adhesive can be applied to the sanitary napkin 20 by means known to the art. For example, gravure coating, slot extrusion, and spray coating, particularly when the adhesive is applied as filaments swirled into a spiral pattern are all suitable. When used with the preferred water soluble adhesive emulsion Airflex 401 described above, gravure coating is particularly preferred.

As also noted above, the absorbent core 28 is joined to one or both of the topsheet 24 and the backsheet 26 using a suitable water soluble adhesive. In the preferred embodiment of the present invention, the sanitary napkin 20, both the topsheet 24 and the backsheet 26 are joined to the absorbent core 28 using the preferred water soluble adhesive emulsion Airflex 401.

If desired, the preferred water sensitive garment attachment adhesive 9216-02 from Findley Adhesives Inc. of Wauwatosa, WI may be applied to the garment surface 26B of the backsheet 26 using slot extrusion or other suitable means and a release liner, as described above, disposed thereon.

When a sanitary napkin 20 of the present invention is assembled as described above, it will readily disperse when exposed to the mild agitation conditions encountered when a conventional toilet is flushed. For example, when such sanitary napkins are evaluated for flushability using the High Loading Protocol described in the TEST METHODS section below they flush in substantially the same manner as a commercially available toilet tissue ((CHARMIN®). These results can be explained by the following model:

1) The water soluble adhesive joining the topsheet, the absorbent core and the backsheet rapidly dissolves allowing the components of the sanitary napkin 20 to separate.

2) This separation exposes protected portions of these components to the water with a resulting decrease in the mechanical strength of these components.

3) The components further disperse into smaller particles that pass through the test apparatus similarly to a toilet tissue (CHARMIN®) control.

Optional Features

Flaps

In an alternative embodiment of the present invention, the sanitary napkin has two flaps each of which are adjacent to and extend laterally from the side edge of the absorbent core in at least the central region. The flaps are configured to drape over the edges of the wearer's panties in the crotch region so that the flaps are disposed between the edges of the wearer's panties and the thighs. The flaps serve at least two purposes. First, the flaps help serve to prevent soiling of the wearer's body and panties by menstrual fluid, preferably by forming a double wall barrier along the edges of the panty. Second, the flaps are preferably provided with attachment means on their garment surface so that the flaps can be folded back under the panty and attached to the garment facing side of the panty. In this way, the flaps serve to keep the sanitary napkin properly positioned in the panty. The flaps can be constructed of various materials including materials similar to the topsheet, backsheet, tissue, or combination of these materials. Further, the flaps may be a separate element attached to the main body of the napkin or can comprise extensions of the topsheet and backsheet (i.e., unitary). A number of sanitary napkins having flaps suitable or adaptable for use with the sanitary napkins of the present invention are disclosed in U.S. 4,687,478, which issued to Van Tilburg on August 18, 1987; U. S. 4,589,876, which issued to Van Tilburg on May 20, 1986; and U.S. 4,608,047, which issued to Mattingly on August 26, 1986.

TEST METHODS

Burst Strength

Overview

The test specimen, held between annular clamps, is subjected to increasing force that is applied by a 0.625 (1.58 cm) inch diameter, polished stainless steel ball. The burst strength is that force that causes the sample to fail. Burst strength may be measured on wet or dry samples.

Apparatus

| | |
|---|---|
| Burst Tester | Intelect-II-STD Tensile Test Instrument, Cat. No. 1451 - 24PGB or the Thwing-Albert Burst Tester are both suitable.<br>Both instruments are available from Thwing-Albert Instrument Co., Philadelphia, PA. The instruments must be equipped with a 2000 g load cell and, if wet burst measurements are to be made, the instruments must be equipped with a load cell shield and a front panel water shield. |
| Conditioned Room | Temperature and humidity should be controlled to remain within the following limits: |
| | Temperature:    73±3_F (23°C±2°C) |
| | Humidity:        50±2% Relative Humidity |
| Paper Cutter | Scissors or other equivalent may be used |
| Pan | For soaking wet burst samples, suitable to sample size |
| Solution | Water for soaking wet burst samples should be equilibrated to the temperature of the conditioned room. |
| Timer | Appropriate for measuring soak time |

Sample preparation

1) Cut the sample to a size appropriate for testing (minimum sample size 4.5 (11.4 cm) in x 4.5 (11.4 cm) in). Prepare a minimum of five samples for each condition to be tested.

2) If wet burst measurements are to be made, place an appropriate number of cut samples into a pan filled with temperature-equilibrated water.

Equipment Setup

1) Set the burst tester up according to the manufacturer's instructions. If an Intelect-II-STD Tensile Test Instrument is to be used the following are appropriate:

Speed: 12.7 centimeters per minute

Break Sensitivity: 20 grams

Peak Load: 2000 grams

2) Calibrate the load cell according to the expected burst strength.

Measurement and Reporting

1) Operate the burst tester according to the manufacturer's instructions to obtain a burst strength measurement for each sample.

2) Record the burst strength for each sample and calculate an average and a standard deviation for the burst strength for each condition.

3) Report the average and standard deviation for each condition to the nearest gram.

Flushability

Overview

As used herein, the term "flushability" is defined as a product's capacity to pass through typical commercially available household toilets and plumbing drainage systems without causing clogging or similar problems that can be directly associated with the physical characteristics of the product. More specifically, catamenial products are evaluated for flushability via relative ease of toilet bowl and trap evacuation and subsequent transport through a simulated plumbing system.

The test procedure is designed to simulate two days of normal toilet usage for a family of 4 (2 men, 2 women). The test employs a flushing sequence to simulate the following conditions: male urination visits, female urination visits (including post urinary drying with tissue), disposal of catamenial product with cleaning using tissue, and bowel movement visits. The amount of tissue to be used for each tissue flush is a normal loading of 2 strips of seven sheets, or the High Loading of 5 strips of seven sheets. The normal loading is based on consumer research regarding typical habits and practices and the high loading is 2.5 times the normal loading. The test is designed to simulate the conditions a product will encounter if it is flushed through a conventional toilet and into a municipal sewer or into a septic tank. Samples are evaluated for: 1) toilet bowl and trap clearance, 2) drain line blockage, and 3) disintegration during flushing.

Apparatus

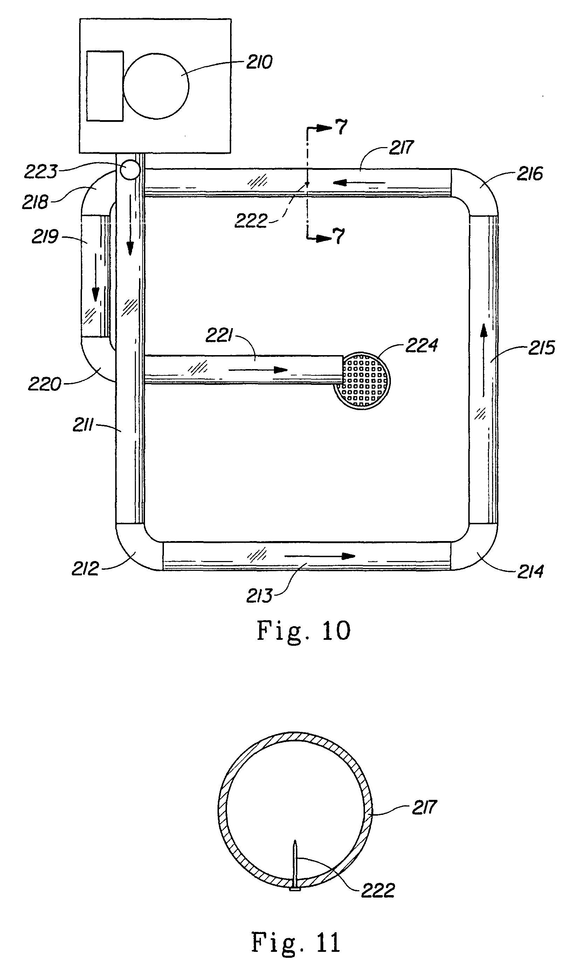

An apparatus suitable for the flushability test is shown in plan view in Figure 10. The apparatus includes:

- a 3.5 gallon (13.2 liter) water saver siphon vortex toilet referred to as 210 (additional toilets can also be attached to the piping layout shown in Figure 10 to evaluate the behavior of test samples using different flushing mechanisms such as commercial, pressure toilets);

- approximately 59 feet (18 meters) of 4 inch (10 cm) id acrylic pipe (As can be seen from Figure 10, the piping is assembled in roughly a square configuration having linear runs 211, 213, 215, 217, 219, 221 approximately 10 feet (3 meters) long);

- a cast iron tee 223 slightly downstream of the toilet 210 that is open to the atmosphere for venting;

- five cast iron ninety degree elbows 212, 214, 216, 218, and 220;

- a snag 222 positioned vertically (Figure 11) approximately 15 feet from the pipe's terminal end and approximately 1 inch (2.5 cm) long; and

- a screen (No. 4 Tyler sieve) to capture solid effluent for evaluation of disintegration.

The apparatus used for this method is set up to be equivalent to ANSI Standard A112.19.2M-1990 for Vitreous China fixtures. The piping is plumbed to provide a drop of 0.25 inch per foot (2 centimeters/meter) of pipe length.

Materials

Control Tissue Product:     CHARMIN®
Synthetic Fecal Material     Prepared according to the method described below

Test Flushing Sequence

The test flushing sequence simulates 2 days of normal toilet usage for a family of 4 (2 men, 2 women; based on consumer habits and practices research). The sequence of 34 total flushes consists of 14 flushes with an empty bowl, 8 flushes with tissue only, 6 flushes with tissue and a catamenial product and 6 flushes with tissue and simulated fecal matter (SFM). When it is used, the SFM is placed in the bowl just prior to the addition of tissue. The SFM loading of 160 g $\pm$ 5 g consists of two 1 inch (2.5 centimeter) x 4 inch (10 centimeter) pieces and one inch (2.5 centimeter) x 2 inch (5 centimeter) piece. Folded tissue strips (or the catamenial product) are placed in the bowl at 10 second intervals. Ten seconds after the final strip or pad is placed into the bowl, the toilet is flushed. The flushing sequence is described below as a series of two routines combined in the following order:

Routine #1 (To be performed 6 times for a total of 30 flushes)

1) Flush With Tissue Only - Take a drain line blockage reading 2 minutes after the water reaches the simulated obstruction, wait 1 additional minute, and move to step 2.

2) Flush With Empty Bowl. Take a drain line blockage reading 2 minutes after the water reaches the snag point and move to step 3.

3) Flush With Tissue and Pad - Take a drain line blockage reading 2 minutes after the water reaches the snag point, wait 1 additional minute, and move to step 4.

4) Flush With Empty Bowl. Take a drain line blockage reading 2 minutes after the water reaches the snag point and move to step 5.

5) Flush With Tissue and Simulated Fecal Mater (SFM). Take a drain line blockage reading 2 minutes after the water reaches the snag point, wait 1 additional minute.

Routine #2 (To be performed 1 time)

1) Flush With Tissue Only - Take a drain line blockage reading 2 minutes after the water reaches the snag point, wait 1 additional minute, and move to step 2.

2) Flush With Empty Bowl. Take a drain line blockage reading 2 minutes after the water reaches the snag point and move to step 3.

3) Flush With Tissue Only - Take a drain line blockage reading 2 minutes after the water reaches the snag point, wait 1 additional minute, and move to step 4.

4) Flush With Empty Bowl. Take a drain line blockage reading 2 minutes after the water reaches the snag point.

Total number of flushes per sequence is 34.

If, at any point in the flushing sequence, the product remains in the bowl or trap after flushing, the tissue and or pad is plunged into the drainage line manually and the flushing sequence will continue. After completion of each trial loading, the drainage pipe will be cleared prior to beginning subsequent testing.

The above-described flushing sequence is repeated three times for each test product and three times for each control product.

Data Reporting

The degree of drain line blockage is determined by measuring the length of water dammed up behind the obstruction. Graduations are marked every 12 inches (30 centimeters) on the drainpipe upstream of the obstruction. Each one foot length that the water is backed up corresponds to 0.25 inch (0.6 centimeter) or 6.25% of blockage at the obstruction point. Test product residues which exit the drainpipe are also collected.

The following data are recorded for each evaluation:

1) Incidence of failure to clear bowl and trap

2) Incidence of labored (difficult), but successful bowl and trap clearing

3) Incidence of product on simulated snag

4) Maximum level (%) of drain line blockage

5) Cumulative level (%) of drain line blockage over 2 days.

Preparation of Synthetic Fecal Material

1. Materials Needed:

• Feclone synthetic fecal matter (900 grams);
   (Available from Siliclone Studio, Valley Forge, PA as product BFPS- 7 dry concentrate )

• Tap water at 100° C (6066 grams)

II. Equipment Needed:

• Mixer (Available from Hobart Corp., Troy, OH as Model A200)

• Extruder (Available from Hobart Corp., Troy, OH as Model 4812)

• Disposable Centrifuge tubes with screw caps (50 ml) (Available from VWR Scientific, Chicago, IL as Catalog No. 21-008-176)

• Water Bath to control temperature to 37° C.

III. Preparation:

1. Pour the 100° C water into the mixing bowl of the mixer and add the dry Feclone concentrate.

2. Mix on low for 1 minute.

3. Mix on medium speed for 2 minutes.

4. After the material is well mixed, transfer to the extruder.

5. Using an ice pick, punch a small hole in the tip of each centrifuge tube.

6. Extrude the Feclone into the centrifuge tubes.

7. Cap the centrifuge tubes and store in the refrigerator.

8. Before using, put the tubes in the water bath at 38° C.

Hydrostatic Head

Overview

The height of a column of water over a sample of material that can be supported with no visual evidence of fluid transport through the sample.

Apparatus

| Conditioned Room | Temperature and humidity should be controlled to remain within the following limits: |
| | |
| | Temperature:    73±3_F (23°C±2°C) |
| | |
| | Humidity:        50±2% Relative Humidity |
| Test Apparatus | The test apparatus is shown in Figure 12 and comprises: |
| | |
| | a water vessel 300 which comprises: |
| | |
| |     1. a glass tube 2.125 inch (0.84 cm) diameter identified as 310; |
| | |
| |     2. a fill tube 312 adapted to deliver water (source not shown) at a controlled rate into the glass tube 310; |
| | |
| |     3. on/off valve 313 for controlling whether water is delivered to the water vessel 300; |
| | |
| |     4. indicia 314 scribed into the surface of the glass tube 310 adapted to allow measurement of the hydrostatic head to an accuracy of ±1 centimeter; |
| | |
| |     5. a male fitting 316 adapted to receive the sample holder 320; and |
| | |
| |     6. an annular rubber gasket 318 positioned beneath the lower end of the glass tube 310; and |
| | |
| | a sample holder 320 which comprises: |
| | |
| |     1. a female fitting 322; |
| | |
| |     2. an annular sample support 324 joined to the lower edge of the female fitting 322; and |
| | |
| |     3. an annular rubber gasket 326. |
| Ring Stand and Clamp | For holding the test apparatus in a vertical position |

Mirror    Placed beneath the sample holder 320 to aid in seeing water penetration of the sample

Method

1. Assemble the apparatus, using a ring stand and clamp to hold the water vessel 300 in a vertical orientation and connecting an adjustable water source to the supply tube 312.

2. Adjust the water temperature to 73°F ± 2°F (23°C±1°C).

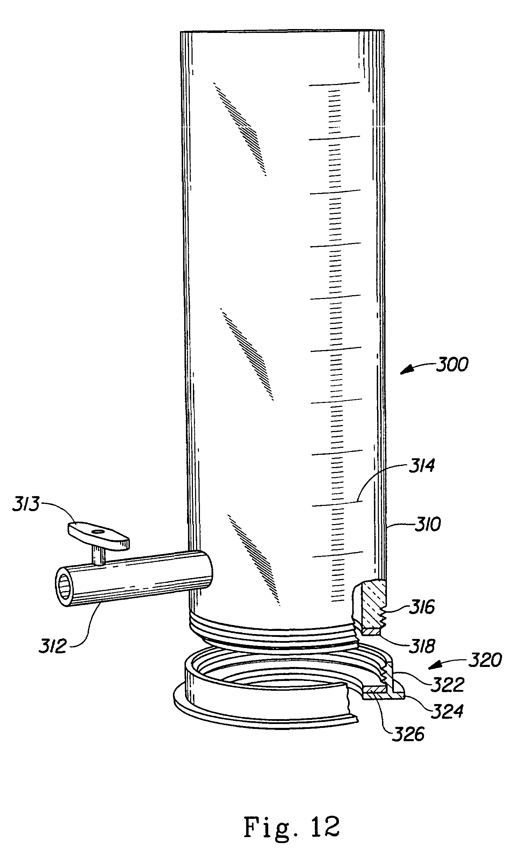

3. Insert a water impervious blank (e. g., polyethylene film) into the sample holder 320, screw the sample holder 320 onto the water vessel 300, open the on/off valve 313, adjust the water flow (adjustment means not shown in Figure 12) so the hydrostatic head rises at a rate of 1 inch per minute ±0.1 inch per minute (2.5 centimeters per minute±0.25 centimeters per minute), and close the on/off valve 313.

4. Die cut a circular sample 2.625 inches in diameter and insert the sample into the sample holder 320. For backsheet samples of the preferred embodiment of the present invention the surface that has been provided with the water resistant resin should be placed facing upward. (The sample should be placed in the conditioned room at least 2 hours prior to testing.).

5. Screw the sample holder 320 onto the water vessel 300 being careful not to crease the sample. Tighten the sample holder only enough to insure that there are no leaks around the sample.

6. Place the mirror under the sample holder 320.

7. Start the water flow into the water vessel 300 by opening the on/off valve 313.

8. Observe the exposed surface of the sample by watching the mirror. Signs of water penetration include beading and spreading of a visible color change on the bottom surface of the sample.

9. Record the height of the column of water when penetration is first observed as the hydrostatic head for the sample.

10. Repeat the measurement 5 times and report the average and standard deviation of the measurements.

Surface Wetness

Overview

Surface wetness is a test designed to measure the amount of liquid which emerges from an absorbent structure, such as the sanitary napkin 20 shown in Figure 1, through a topsheet to cause wetness on the surface of the topsheet. The amount of moisture drawn through the topsheet is termed "surface wetness" and serves as an estimate of how dry the wearer's skin would remain if placed in contact with the absorbent structure.

Method

The test comprises wetting a 4 inch (10 centimeter) x 4 inch (10 centimeter) sample of a topsheet material while superposed, body surface 24A facing up, on a standardized absorbent element preferably comprising a layer of airlaid comminuted wood pulp fibers enveloped between a pair of wet strength tissue plies with a simulated urine solution (available from Jayco Pharmaceuticals, Mehcanicsburg, PA). The simulated urine solution (4.0±0.3 ml) is delivered to the surface of the sample using a syringe pump. A uniform pressure loading of 0. 25 psi (1.7kPa). is applied to each sample while the simulated urine is being delivered so that the fluid is uniformly distributed throughout the sample. After all of the simulated urine has been delivered, the wetted sample is allowed to sit undisturbed for 5±0.5 minutes. The sample is covered with polyethylene film to minimize evaporation while the sample is sitting. The pressure is momentarily removed. A preweighed sample of filter paper (7 plies) approximately 5 inches (12 centimeters) x 5 inches (12 centimeters) is inserted over the uppermost surface of the topsheet of the absorbent sample (Suitable filter paper is available from Ahlstrom Filtration Company of Mt. Holly springs, PA as Paper No 632). Sufficient weight to apply a predetermined pressure loading of 0.5 psi (3.4 kPa) is applied to the sample for a period of 15±1 seconds and removed. The filter paper is then removed and reweighed The amount of fluid absorbed by the filter paper is termed the "surface wetness" of the sample. Results are expressed in grams of fluid absorbed by the filter paper. As should thus be apparent, a lower "surface wetness" number is indicative of dryer surface feel.

Critical Surface Tension

The method described in TAPPI (Technical Association of the Pulp and Paper Industry) method T 698 pm-83, "Determination of Wetting Tension of Polyethylene and Polypropylene Films (modified visking analytical technique)" was used substantially as described therein with the exception that a commercially available series of known surface tension liquids (available from Corotec Corporation, Collinsville, CT) was used instead of the mixtures described in the TAPPI method and brushes, as supplied with the Corotec series were used instead of the cotton swabs.

**Claims**

1. A water dispersible and flushable absorbent article (20), the absorbent article comprising: a liquid pervious topsheet (24), a liquid impervious backsheet (26) disposed beneath said topsheet and an absorbent core (28) disposed between said topsheet and said backsheet **characterized by**

   said topsheet comprising a first fibrous assembly having a temporary wet strength resin incorporated therein, wherein portions of a body surface of said topsheet have preferably been provided with a first resinous material,

   said backsheet comprising a second fibrous assembly having a temporary wet strength resin incorporated therein, said backsheet being coated on the topsheet facing surface with a second resinous material wherein said second resinous material is water resistant,

   and said topsheet and said backsheet being joined using a water soluble adhesive in at least an area of peripheral bonding to encapsulate said absorbent core there between.

2. An absorbent article (20) according to Claim 1 wherein said first resinous material comprises fibrils of a water resistant resinous material.

3. An absorbent article (20) according to Claims 1 or 2 wherein said portions of said body surface provide a plurality of regions of comparatively low surface energy, which define surface energy gradients where they interface with higher surface energy web surfaces.

4. An absorbent article (20) according to any of the above claims wherein said absorbent article further comprises a means for attaching said absorbent article to a wearer's undergarment.

5. An absorbent article (20) according to any of the above claims wherein said backsheet (20) has a body surface and a garment surface and said second resinous material enables said backsheet to resist a hydrostatic head of at least about 18 cm and provides said body surface of said backsheet with a critical surface tension of greater than about 34 dynes per centimeter.

6. An absorbent article (20) according to any of the above claims wherein said fibrils are provided at a density of between about 3000 fibrils per square inch (465 fibrils per square centimeter) and about 5000 fibrils per square inch (775 fibrils per square centimeter).

7. An absorbent article (20) according to any of the above claims wherein said absorbent article further has longitudinal and transverse centerlines (L, T) and a pair of longitudinally oriented sides (23) and said sanitary further comprises at least one flap which is joined to one of said sides and extends transversely outwardly therefrom.

**Patentansprüche**

1. In Wasser dispersibler und wegspülbarer, absorbierender Artikel (20), wobei der absorbierende Artikel umfaßt: eine flüssigkeitsdurchlässige Oberschicht (24), eine unterhalb der Oberschicht angeordnete flüssigkeitsundurchlässige Unterschicht (26) und einen zwischen der Oberschicht und der Unterschicht angeordneten absorbierenden Kern (28), **gekennzeichnet durch**

die Oberschicht, welche eine erste Faseranordnung mit einem darin eingebauten, temporär naßfesten Harz aufweist, wobei Bereiche einer körperseitigen Oberfläche der Oberschicht vorzugsweise mit einem ersten harzartigen Material versehen sind,

die Unterschicht, welche eine zweite Faseranordnung mit einem darin eingebauten, temporär naßfesten Harz aufweist, wobei die Unterschicht auf der der Oberschicht entgegen gerichteten Seite mit einem zweiten harzartigen Material beschichtet ist, wobei das zweite harzartige Material wasserresistent ist,

und wobei die Oberschicht und die Unterschicht unter Verwendung eines wasserlöslichen Haftmittels in wenigstens einer Fläche einer umfänglichen Bindung miteinander verbunden sind, um den absorbierenden Kern zwischen sich einzukapseln.

2. Absorbierender Artikel (20) nach Anspruch 1, in welchem das erste harzartige Material Fibrillen eines wasserresistenten harzartigen Materials umfaßt.

3. Absorbierender Artikel (20) nach Anspruch 1 oder 2, in welchem die Bereiche der körperseitigen Oberfläche eine Mehrzahl von Regionen vergleichsweise geringer Oberflächenenergie schaffen, welche dort Gradienten der Oberflächenenergie definieren, wo sie mit Bahnoberflächen höherer Oberflächenenergie zusammen treffen.

4. Absorbierender Artikel (20) nach einem der obigen Ansprüche, in welchem der absorbierende Artikel ferner ein Mittel zum Anbringen des absorbierenden Artikels an einer Unterwäsche eines Trägers umfaßt.

5. Absorbierender Artikel (20) nach einem der obigen Ansprüche, in welchem die Unterschicht (20) eine körperseitige Oberfläche und eine wäscheseitige Oberfläche hat und das zweite harzartige Material die Unterschicht in die Lage versetzt, eine hydrostatischen Spitze von wenigstens etwa 18 cm und der körperseitigen Oberfläche der Unterschicht eine kritische Oberflächenspannung von größer als etwa 34 Dyn pro Zentimeter zu verleihen.

6. Absorbierender Artikel (20) nach einem der obigen Ansprüche, in welchem die Fibrillen in einer Dichte von zwischen etwa 3000 Fibrillen pro Quadratinch (465 Fibrillen pro Quadratzentimeter) und etwa 5000 Fibrillen pro Quadratinch (775 Fibrillen pro Quadratzentimeter) vorgesehen sind.

7. Absorbierender Artikel (20) nach einem der obigen Ansprüche, in welchem der absorbierende Artikel ferner eine längs verlaufende und eine quer verlaufende Mittellinie (L, T) und ein Paar längs orientierter Seiten (23) hat und die Binde ferner wenigstens eine Klappe aufweist, welche mit einer der Seiten verbunden ist und sich von dieser quer nach außen erstreckt.

**Revendications**

1. Article absorbant (20) éliminable à la chasse et dispersible dans l'eau, l'article absorbant comprenant : une feuille de dessus perméable aux liquides (24), une feuille de fond imperméable aux liquides (26) disposée en dessous de ladite feuille de dessus, et une âme absorbante (28) disposée entre ladite feuille de dessus et ladite feuille de fond, **caractérisé en ce que** :

ladite feuille de dessus comprend un premier ensemble fibreux dans lequel est incorporée une résine ayant une résistance temporaire à l'état humide, des parties d'une surface côté corps de ladite feuille de dessus étant de préférence pourvues d'une première matière résineuse,
ladite feuille de fond comprend un deuxième ensemble fibreux dans lequel est incorporée une résine ayant une résistance temporaire à l'état humide, ladite feuille de fond étant enduite d'une deuxième matière résineuse sur sa surface en regard de la feuille de dessus, ladite deuxième matière résineuse étant résistante à l'eau, et
ladite feuille de dessus et ladite feuille de fond sont réunies à l'aide d'un adhésif soluble dans l'eau, dans au moins une région de liaison périphérique, pour encapsuler ladite âme absorbante entre elles.

2. Article absorbant (20) selon la revendication 1, dans lequel ladite première matière résineuse comprend des fibrilles d'une matière résineuse résistante à l'eau.

3. Article absorbant (20) selon les revendications 1 ou 2, dans lequel lesdites parties de ladite surface côté corps forment une pluralité de régions ayant une énergie superficielle relativement faible, qui définissent des gradients d'énergie superficielle au niveau desquels elles sont reliées à des surfaces de nappe ayant une plus grande énergie superficielle.

4. Article absorbant (20) selon l'une quelconque des revendications précédentes, dans lequel ledit article absorbant comprend des moyens servant à attacher ledit article absorbant aux sous-vêtements d'une utilisatrice.

5. Article absorbant (20) selon l'une quelconque des revendications précédentes, dans lequel ladite feuille de fond (20) a une surface côté corps et une surface côté vêtement, et ladite deuxième matière résineuse permet à ladite feuille de fond de résister à une pression hydrostatique d'au moins environ 18 cm et confère à ladite surface côté corps de ladite feuille de fond une tension superficielle critique supérieure à environ 34 dynes/cm.

6. Article absorbant (20) selon l'une quelconque des revendications précédentes, dans lequel lesdites fibrilles sont prévues en une densité comprise entre environ 465 fibrilles/cm$^2$ (3000 fibrilles par pouce carré) et environ 775 fibrilles/cm$^2$ (5000 fibrilles par pouce carré).

7. Article absorbant (20) selon l'une quelconque des revendications précédentes, dans lequel ledit article absorbant a, en outre, des lignes médianes longitudinale et transversale (L, T) et une paire de côtés orientés dans la direction longitudinale (23), et ledit article hygiénique comprend, en outre, au moins un rabat qui est réuni à l'un desdits côtés et qui s'étend dans la direction transversale, vers l'extérieur, à partir de celui-ci.

Fig. 1

Fig. 2

Fig. 3

Fig. 4

6

52

50

24

Fig. 5

56

55

54

57

Fig. 9

54

50

52

Fig. 6

54

52

Fig. 7

54

52

Fig. 8

Fig. 10

Fig. 11

Fig. 12